# EUROPEAN PATENT SPECIFICATION

(11) **EP 4 103 256 B1**
(45) Date of publication and mention of the grant of the patent: **29.10.2025**
(21) Application number: 21715691.8
(22) Date of filing: 12.02.2021
(51) Int. Cl.: A61M 25/00, A61M 25/10

(54) **VASCULAR TREATMENT SYSTEMS**
BEHANDLUNGSSYSTEME FÜR BLUTGEFÄSSE
SYSTÈMES DE TRAITEMENT VASCULAIRE

(30) Priority: 13.02.2020 US 202062976342 P
(43) Date of publication of application: 21.12.2022
(73) Proprietor: The Foundry, LLC, Menlo Park, CA 94025 (US)
(72) Inventor: GIFFORD, III, Hanson, S., Woodside, CA 94062 (US); GIFFORD, Edward, DeWitt, Glastonbury, CT 06033 (US); LEVERING, Vrad, W., Smithville, TX 78957 (US)
(74) Representative: Forresters IP LLP
(86) International application number: PCT/US2021/018047
(87) International publication number: WO 2021/163635

(56) References cited:
- WO-A1-96/15825
- US-A- 5 344 402
- US-A1- 2004 019 322
- US-A1- 2011 264 039
- US-A1- 2012 209 375

## Description

### CROSS-REFERENCE TO RELATED APPLICATIONS

The present application claims the benefit of priority to U.S. Patent No. 62/976,342, filed February 13, 2020.

PCT Application No. PCT/US19/46070, filed August 9, 2019, and U.S. Provisional Application No. 62/717,752, filed August 10, 2018.

### TECHNICAL FIELD

The present technology is directed to treatment devices, systems, and methods for treating cardiac disease. In particular, the present technology relates to devices for treating blood vessels and associated systems and methods of use.

### BACKGROUND

There are many situations in interventional vascular procedures where there is a need to inflate a balloon in a vessel while maintaining perfusion through that vessel. For example, when delivering a balloon-expandable stent in a coronary artery, it is preferable to maintain blood flow through the artery to avoid ischemic damage to the myocardium perfused by that artery. One device commonly used to maintain blood flow is a perfusion balloon catheter. These catheters typically have a relatively large central guidewire lumen with holes through the catheter sidewall into the lumen just proximal to the balloon. This allows blood to flow through the side holes, into the guidewire lumen, and out the distal end of the catheter to maintain perfusion when the balloon is inflated and occluding the vessel.

However, these perfusion balloons are typically only used in smaller vessels where a relatively small perfusion lumen is sufficient, and the catheter can be made with a perfusion lumen of a fixed size, for example a lumen of less than 2 mm in diameter. In larger vessels such as the aorta, maintaining adequate distal perfusion without a high pressure gradient through the perfusion lumen requires a much larger lumen.

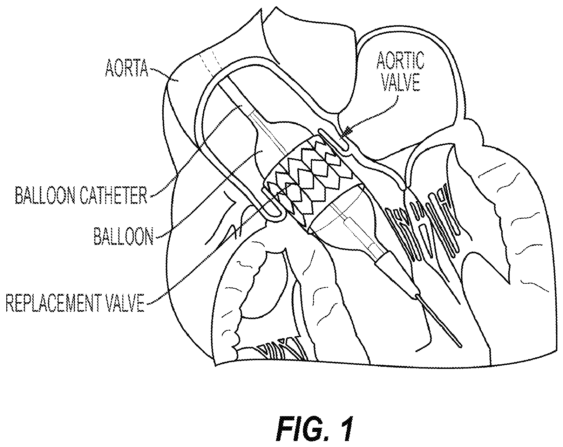

Two common interventional procedures that utilize balloon expansion are (a) balloon valvuloplasty of the aortic valve, and (b) catheter-based delivery of balloon-expandable replacement aortic valves (commonly referred to as "transcatheter aortic valve replacement" or "TAVR"). FIG. 1 shows the delivery of a replacement aortic valve on a conventional balloon catheter. Conventional practice is to inflate the balloon to a large diameter (about 20-30 mm) at a very high pressure, blocking all flow to the systemic circulation. It also prevents any blood from leaving the left ventricle, which can lead to a dangerous acute expansion of the ventricle. In order to prevent this dangerous expansion of the ventricle, a temporary pacing catheter is placed in the heart and the heart is typically paced at a very high rate (~200 beats per minute) which prevents it from filling between heartbeats. Such rapid ventricular pacing may cause myocardial ischemia, malignant arrhythmias, low output, reduced cerebral oxygen saturation, and/or increased procedure time and risk of stroke. To avoid or reduce the likelihood of these dangers, the valvuloplasty or TAVR balloon is typically inflated for less than a minute.

In a typical balloon catheter, the balloon is formed from a single extrusion which is expanded into the desired balloon shape and welded or bonded to the shaft of a catheter. The sidewall of the catheter is cut to create an opening to connect an inflation lumen running through the catheter shaft to the interior of the balloon. In most clinical applications in which a vessel or valve is being dilated, the outer surface of the balloon is rounded (i.e., has a functionally circular cross-sectional shape) so as to apply a relatively even radial force against the apposing tissue. It is difficult to locate a perfusion lumen at the outer circumference of the balloon while maintaining this rounded/circular shape and providing even radially outward force.

An existing approach to the foregoing challenge of creating a large perfusion lumen in larger balloon catheters is the TRUE^{®} Flow Valvuloplasty Perfusion Catheter (C.R. Bard/Becton Dickenson). The TRUE^{®} Flow device has several smaller balloons arranged around the periphery of a central lumen and surrounded by a fiber-based shell. When inflated, the balloons hold the central lumen open. However, this approach limits the effective pressure which can be applied to the circumference of the balloon, even when these smaller balloons are inflated to a higher pressure. More importantly, the use of multiple balloons employs a large amount of material which increases a deflated diameter of the device and makes delivery of the device through a delivery sheath more difficult. This is especially true in the case of a TAVR balloon that has the additional bulk of the prosthetic valve. Document WO96/15825A1 relates to a dilatation catheter for percutaneous transluminal coronary angioplasty having perfusion capabilities.

Therefore, there remains a need for improved balloons for interventional procedures, especially within the field of interventional cardiology.

### SUMMARY

It is noted that the scope of protection of the current invention is defined by the appended claims.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1 depicts a conventional balloon catheter and replacement valve positioned at the aortic valve during a typical TAVR procedure.
FIG. 2A is a side view of a distal portion of a treatment system of the present technology, shown in a collapsed state.
FIGS. 2B and 2C are front and rear isometric views, respectively, of a distal portion of the treatment system of the present technology, shown in an expanded state.
FIG. 2D is a cross-sectional side view of the treatment system of the present technology in an expanded state.
FIG. 2E is a cross-sectional end view of the treatment system of the present technology taken along line 2E-2E in FIG. 2D.
FIGS. 3A and 3B are side and isometric views, respectively, of a mold for forming an expandable element in accordance with the present technology.
FIG. 4A is a side view of a reinforcing element of the present technology in a rolled-up, collapsed state.
FIG. 4B is a top view of a reinforcing element of the present technology in a laid-flat configuration.
FIG. 5 is an isometric view of a treatment element of the present technology.
FIG. 6A is an enlarged, isolated view of a portion of the reinforcing element of FIGS. 4A and 4B while the reinforcing element is in a collapsed state.
FIG. 6B is an enlarged, isolated view of the portion of the reinforcing element shown in FIG. 6A as the reinforcing element moves from the collapsed state of FIG. 6A to an expanded state.
FIG. 7 is a top view of a reinforcing element of the present technology in a laid-flat configuration.
FIG. 8A is a side view of a distal portion of a treatment system of the present technology.
FIG. 8B is a rear isometric view of a distal portion of a treatment system of the present technology.
FIG. 9A is a schematic side view of a treatment system of the present technology, shown in an expanded configuration.
FIG. 9B is a cross-sectional end view taken along line 9B-9B in FIG. 9A.
FIG. 9C is a cross-sectional end view taken along line 9C-9C in FIG. 9A.
FIGS. 10A and 10B illustrate different reinforcing elements in accordance with the present technology.
FIG. 11 is a schematic, cross-sectional side view of a treatment system of the present technology, shown in an expanded configuration.
FIGS. 12A-12D depict the foreshortening of the reinforcing element in response to relative movement of the elongated members of the treatment system.
FIGS. 13A and 13B are schematic, cross-sectional side views of a distal portion of a treatment system configured in accordance with the present technology, shown in a collapsed configuration and an expanded configuration, respectively.
FIGS. 14A and 14B are schematic, cross-sectional side views of a distal portion of a treatment system configured in accordance with the present technology, shown in a collapsed configuration and an expanded configuration, respectively.
FIG. 15 is a schematic, cross-sectional side view of a distal portion of a treatment system configured in accordance with the present technology, shown in an expanded configuration, respectively.

### DETAILED DESCRIPTION

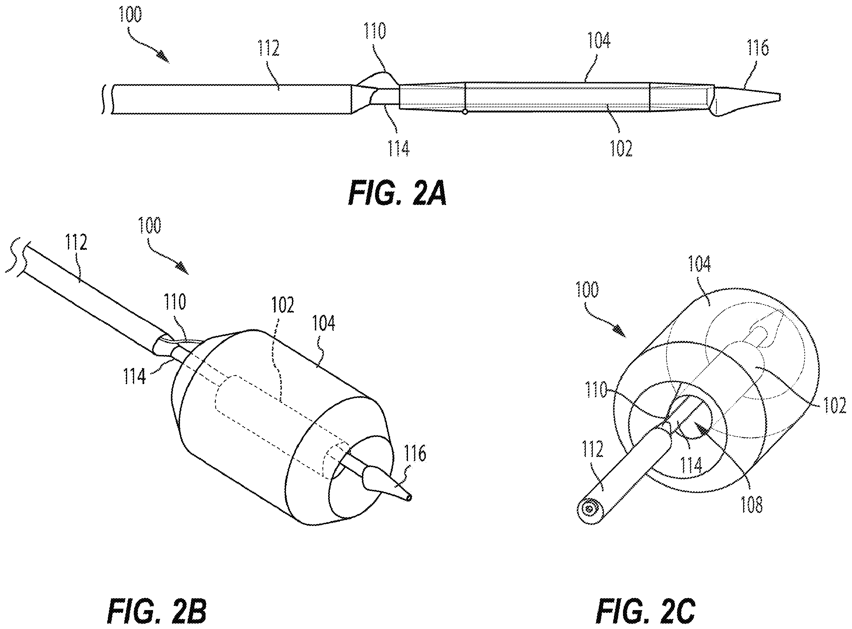

Specific details of several embodiments of the technology are described below with reference to FIGS. 2A-15. Although many of the embodiments are described below with respect to devices, systems, and methods for percutaneous replacement of a native aortic valve, other applications and other embodiments in addition to those described herein are within the scope of the technology, such as devices, systems, and methods for performing a balloon valvuloplasty, for dilating aortic strictures or other narrowings in the circulatory system, and devices, systems, and methods for percutaneous replacement of a native mitral valve, a native tricuspid valve, and/or a native pulmonic valve. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described below with reference to FIGS. 2A-15.

With regard to the terms "distal" and "proximal" within this description, unless otherwise specified, the terms can reference a relative position of the portions of an interventional device such as a prosthetic valve device and/or an associated delivery device with reference to an operator and/or a location in the vasculature or heart. For example, in referring to a delivery system including the perfusion devices described herein, "proximal" can refer to a position closer to the operator of the device or an incision into the vasculature, and "distal" can refer to a position that is more distant from the operator of the device or further from the incision along the vasculature (e.g., the end of the catheter).

FIG. 2A is a side view of a distal portion of a treatment system 100 of the present technology, shown with a treatment element 101 in a collapsed state. FIGS. 2B and 2C are front and rear isometric views, respectively, of the distal portion of the treatment system 100 with the treatment element 101 in an expanded state. As used herein with reference to the treatment system 100, "expanded configuration" and "collapsed configuration" refer to the configuration of the treatment system 100 when the treatment element 101 is expanded or collapsed, respectively. Likewise, the use of "deployed" with respect to the treatment element 101 or treatment system 100 refers to a configuration in which the treatment element 101 is in a fully or partially expanded configuration.

The treatment system 100 is configured to position and deploy the treatment element 101 at a treatment site within a blood flow passage of a patient to provide a perfusion lumen while the expandable element 104 is deployed during an interventional procedure. As used herein, "blood flow passage" refers to any anatomical structure through which blood regularly flows. Examples of blood flow passages include a native annulus (in the heart or anywhere in the vasculature), a heart chamber, a blood vessel, and other body lumens. The treatment element 101 may be delivered in the collapsed state through a guide catheter to the treatment site within the blood flow passage. When treating a blood flow passage at or within the heart, the treatment system 100 may deliver the treatment element 100 to the heart via transfemoral, transcaval, trans-aortic, trans-venous, trans-atrial, transaxilliary/subclavian, or trans-apical approaches, antegrade or retrograde to the direction of blood flow, and may include trans-septal crossing from the right atrium to the left atrium.

The treatment systems 100 of the present technology may be used in any medical procedure that employs balloon expansion within a blood flow passage, regardless of whether the balloon (or other expandable element) is expanded directly into contact with the surrounding passage tissue or indirectly with another structure positioned between the balloon and the passage tissue (such as a stent, stent-graft, or prosthetic heart valve apparatus). The treatment systems 100 disclosed herein, for example, provide a reinforced perfusion lumen through a balloon while the balloon is expanded within a native valve annulus to widen the native valve opening during a valvuloplasty or to position a prosthetic valve during a TAVR procedure. The treatment systems 100 of the present technology may be used for delivery of repair or replacement devices to any of the four major cardiac valves (aortic, mitral, tricuspid, and pulmonic), as well as dilation of strictures or stents in the aorta or any of the great vessels or other blood vessels, and others. It might also be used for temporary sealing of aortic ruptures, uncontrolled bleeding sites, aortic dissections, or other areas where sealing of the vessel while sustaining perfusion is desired. Additionally, the treatment systems 100 of the present technology may be utilized in trans-septal as well as fenestrated aortic applications, where target vessel or lesion access requires navigation through tortuous anatomy. In such scenarios, an outer, large diameter balloon (such as the expandable element of the present technology) allows for fixation adjacent to the target lesion or vessel. In some cases, treatment systems for additional stent or balloon dilation could be delivered through the central lumen of the expandable element of the present technology.

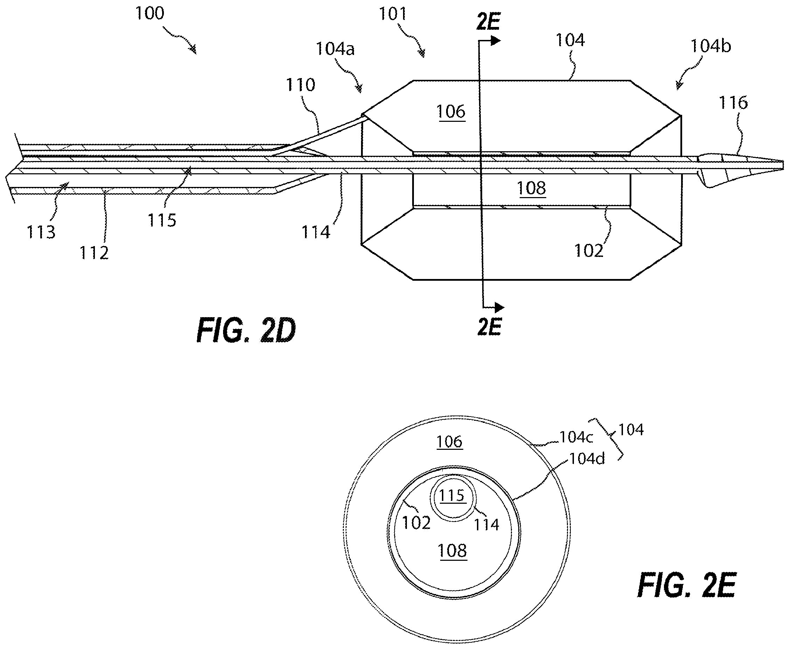

As shown in FIGS. 2A-2C and the cross-sectional views of FIGS. 2D and 2E, the treatment system 100 may comprise the treatment element 101 and one or more elongated members configured to carry or otherwise functionally support the treatment element 101 within the blood flow passage. The treatment element 101 comprises an inner reinforcing element 102 (e.g., a stent) and an outer expandable element 104 (e.g., a balloon) positioned around the reinforcing element 102. As described in greater detail below, the reinforcing element 102 is coupled to the expandable element 104 such that inflation of the expandable element 104 causes radial expansion of the reinforcing element 102, thereby creating a perfusion lumen 108 through the deployed treatment element 101 at the treatment site that allows blood to flow through the passage while the expandable element 104 is deployed during the interventional procedure. If the expandable element 104 were to be inflated (or otherwise expanded) without deploying the inner reinforcing element 102, the pressure in the expandable element 104 may collapse the perfusion lumen 108. As such, the reinforcing element 102 provides a radially outward force against an inner surface of the expandable element 104 to maintain a perfusion lumen through the expandable element 104 while it is inflated.

In some embodiments, the expandable element 104 comprises an inflatable element, such as a balloon. The expandable element 104 can comprise one or more substantially fluid-impermeable materials commonly used for balloons used in interventional procedures, such as polyethylene, polyolefin, polyurethane, nylon (polyamide), polyethylene terephthalate (PET or polyester), and/or other suitable polymers. In several examples of the technology, the expandable element 104 may comprise a composite structure formed of one or more polymer(s) and reinforcing fibers, such as Kevlar, carbon-fiber, spectra or other high molecular weight polyethylenes, or other fibers in any orientation.

As best shown in the cross-sectional view of FIG. 2E, the expandable element 104 may comprise an elongated, annular balloon formed of an outer wall 104c and an inner wall 104d that together enclose an interior region 106. The interior region 106 may be configured to receive a fluid to inflate or otherwise expand the expandable element 104. The outer and inner walls 104c and 104d of the expandable element 104 may be substantially impermeable to fluid such that delivery of a fluid to the interior region 106 when the expandable element 104 is in its collapsed configuration forces the outer wall 104c to move radially away from the inner wall 104d, thereby causing the expandable element 104 to radially expand. It may also become longer, or it may foreshorten with respect to the elongate member as it expands radially. As can be seen in comparing the collapsed configuration of the expandable element 104 (FIG. 2A) to its expanded configuration (FIGS. 2B-2E), the expandable element 104 may be slightly longer in its collapsed configuration than in its expanded configuration.

In some embodiments, such as that shown in FIGS. 2A-2E, the expandable element 104 may be formed of an inverted tube such that the expandable element 104 comprises a seam at a proximal or first longitudinal end 104a (FIG. 2D) and a fold (and no seam) at a distal or second longitudinal end 104b (FIG. 2D). In some embodiments, the expandable element 104 comprises a fold (and no seam) at the proximal or first longitudinal end 104a (FIG. 2D) and a seam at a distal or second longitudinal end 104b (FIG. 2D). In any case, the fold may comprise the inverted portion of the tube, and the seam may comprise where the two longitudinal ends of the tube meet. Depending on the material used for the expandable element 104 (for example, if using polyurethane), the seam may be welded, or it can also be bonded using flexible bonding agents such as UV-curable urethane methacrylates. The inflation shaft (such as the first elongated member 110, described below) can be made of the same or a similar material and welded or bonded into the seam. It may be preferable to reduce or otherwise minimize any seams, joints or welds in the expandable element 104 to reduce the likelihood of having weakened sections of the expandable element 104 that are less capable of withstanding high pressures.

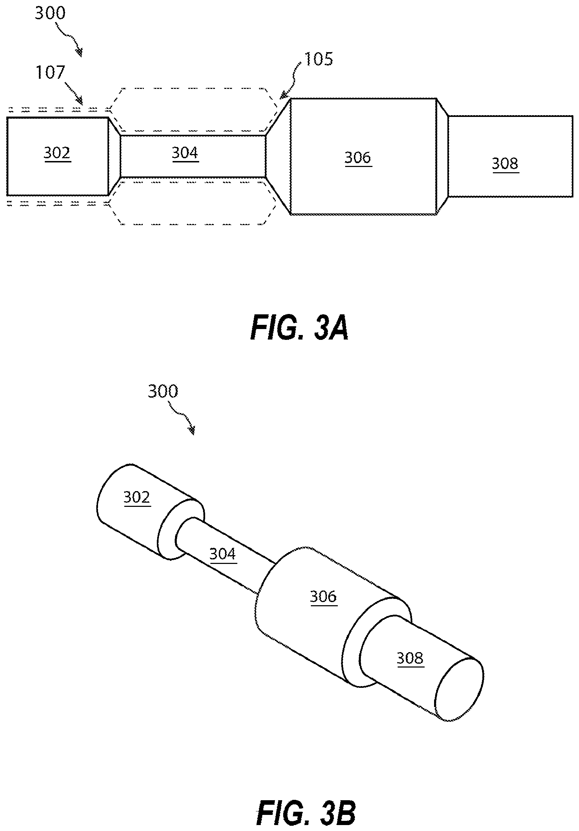

FIGS. 3A and 3B show an example mold 300 for forming an expandable element 104 of the present technology. The mold 300 can have a first portion 302, a second portion 304, a third portion 306, and a fourth portion 308 along its length that result in analogous sections of the resultant balloon. Adjacent portions can have a tapered transition portion therebetween. In some embodiments, a diameter of the second portion 304 may be less than the individual diameters of the first, third, and fourth portions 302, 306, 308, and can reflect the inner diameter of the lumen of the expandable element 104. The dashed line shows an example of a tube that has been placed on the mold, blown to fit the mold shape, and then folded over itself such that the resulting structure has a fold 105 at one end and a seam 107 at the other. A tube (e.g., elongated member 110) can be inserted into the seam 107 to define an inflation lumen. It will be appreciated that the expandable element 104 may have other shapes than that shown in the drawings, and the mold 300 may have other shapes. When in its expanded configuration, the outer wall of the expandable element 104 may have a contour comprised of generally linear sections (as shown in the drawings), or all or certain portions of the expandable element's outer wall may have a more rounded contour than that which is depicted in the drawings.

In some embodiments, the expandable element 104 may comprise a balloon that is everted at both ends so that the two ends overlap along the interior diameter of the balloon. In this configuration, the resulting seam may be disposed at an intermediate portion of the balloon rather than at a proximal or distal end. For example, the seam may be disposed along a central portion of the balloon along the radially inward surface. The two overlapping ends may be bonded together using adhesive, for example. The inflation lumen may be inserted between these two overlapping ends and bonded into the balloon when the ends are bonded together.

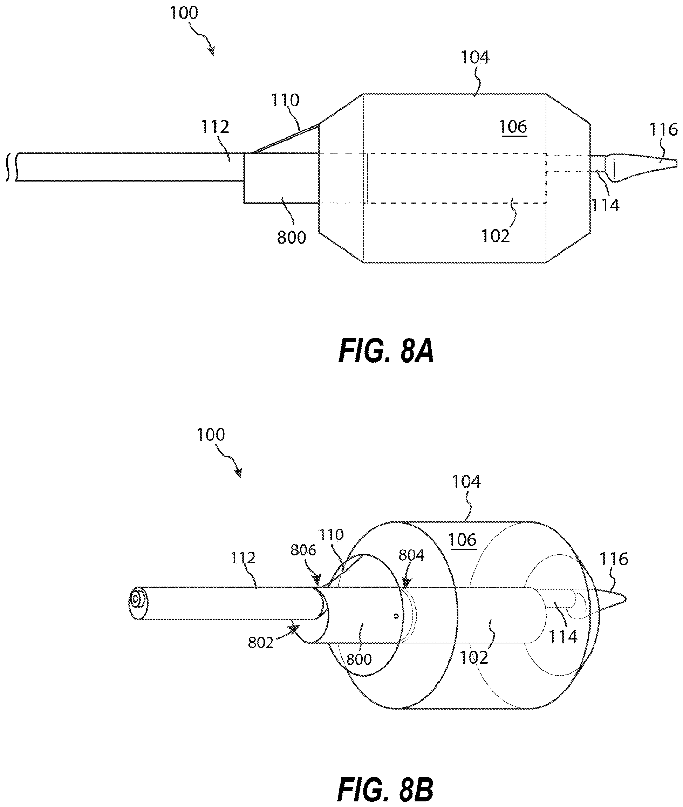

In some aspects of the present technology, the expandable element 104 may be constructed using two discrete layers: (a) a first material or layer for the inner wall that may contact the reinforcing element 102 (i.e., facing the lumen 108) and optionally a guidewire shaft (such as the second elongated member 114, detailed below), and (b) one for the outer wall. In some embodiments where the treatment element 101 includes a valve (such as that described with respect to FIGS. 8A and 8B), the outer wall should be connected to the valve so that there is no path for leakage between the expandable element and the valve. The two materials or layers can be bonded at two seams, distal and proximal, with an inflation lumen welded or bonded into the one of the seams.

In some embodiments, the expandable element 104 of the present technology may have one or more of the following features: a very low deflated or collapsed profile to minimize the device diameter on introduction and removal; a relatively large inflated or expanded profile (e.g., about 20 mm to about 30 mm, for example to allow dilation of large valves or vessels such as the aortic valve or the aorta); a large perfusion lumen when inflated or expanded to reduce or minimize any pressure gradient across the expandable element 104 (for example, a diameter of the perfusion lumen 108 when the treatment element 101 is in an expanded state is sufficient to minimize any pressure gradient of blood flowing through the perfusion lumen and/or a diameter of the perfusion lumen is at least one-third of a diameter of the blood flow passage); and capability of withstanding high inflation pressures (e.g., at least about 30 to about 105 psi (about 2 to about 7 atmospheres)) to effectively dilate stenosed valves or aortic strictures.

The expandable element 104 of the present technology provides several advantages over conventional balloon-expandable systems. For example, unlike conventional devices, the expandable element 104 is not formed of a sidewall bonded to a fixed-diameter catheter shaft. Rather, the expandable element 104 has an elongated, annular or toroidal shape with an exterior surface that has both a luminal-facing (e.g., radially inward-facing) portion and a radially outward-facing portion. Moreover, the elongated, annular expandable element 104 of the present technology has a shorter length without necessitating a steeper transition angle from the large diameter since much of the transition is "reversed" by the eversion. The shorter expandable element 104 improves pushability and maneuverability of the treatment system 100 when navigating the more tortuous anatomy.

Referring still to FIGS. 2A-2E, in addition to the treatment element 101, the treatment system 100 may comprise one or more elongated members. For example, the treatment system 100 may comprise a first elongated member 110 defining a first lumen therethrough (not visible in the drawings), a second elongated member 114 defining a second lumen 115 therethrough, which might be used to deliver a guidewire in advance of the rest of the system, and a third elongated member 112 defining a third lumen 113 therethrough. The first elongated member 110 may extend between a proximal end portion (not shown) and a distal end portion that is fluidically coupled to the interior region 106 of the expandable element 104. For example, a distal end portion of the first elongated member 110 may be inserted into and/or placed into fluid communication with the interior region 106 of the expandable element 104 via a seam between the outer and inner walls 104c, 104d (FIG. 2E) at a proximal end portion of the treatment element 101. The first elongated member 110 may be configured to deliver a fluid (e.g., water, saline, a radiopaque solution, air) to the expandable element 104 to expand the expandable element 104.

The second elongated member 114 may extend between a proximal end portion (not shown) and a distal end portion located at or near the treatment element 101. The second elongated member 114 can be a generally tubular shaft defining a lumen 115 therethrough. In some embodiments, the second elongated member 114 is configured to slidably receive a guidewire for atraumatic guidance of the catheter to the appropriate location. The second elongated member 114 may extend through the entire length of the perfusion lumen 108 and terminate distal to a distal end of the treatment element 101, the expandable element 104, and/or the reinforcing element 102. In some embodiments, the second elongated member 114 may terminate at a location that is longitudinally aligned with or proximal of a distal end of the treatment element 101, the expandable element 104, and/or the reinforcing element 102. In some embodiments, the second elongated member 114 may have an atraumatic distal tip at its distal end, as shown in FIGS. 2A-2D.

The second elongated member 114 may be coupled to the reinforcing element 102 such that the reinforcing element 102 is free to radially contract and expand and/or longitudinally contract and expand. For example, the second elongated member 114 may be coupled to the reinforcing element 102 at a single longitudinal location that does not hinder expansion and contraction of the reinforcing element. In some embodiments, the second elongated member 114 may be coupled to the reinforcing element 102 at multiple longitudinal locations along the reinforcing element 102, each of which is not subject to foreshortening (as described in greater detail below).

The third elongated member 112 may comprise a generally tubular shaft defining a lumen 113 therethrough. The third elongated member 112 may extend from a proximal portion (not shown) to a distal portion that terminates at a location longitudinal aligned with or proximal of the treatment element 101, the expandable element 104, and/or the reinforcing element 102. In some embodiments, the third elongated member 112 terminates at a location longitudinally aligned with or distal to a proximal end of the treatment element 101, the expandable element 104, and/or the reinforcing element 102. In some embodiments, the third elongated member 112 extends through the entire length of the treatment element 101, the expandable element 104, and/or the reinforcing element 102.

The third elongated member 112 may receive one or more elongated members (including tubular shafts) through its lumen 113. For example, as shown in FIGS. 2A-2E, the first and second elongated members 110 and 114 extend through the lumen 113 of the third elongated member 112. The third elongated member 112 adds column strength to the treatment system 100, thereby improving pushability.

In some embodiments, the treatment system 100 may have more or fewer elongated members or lumens than that depicted in FIGS. 2A-2E.

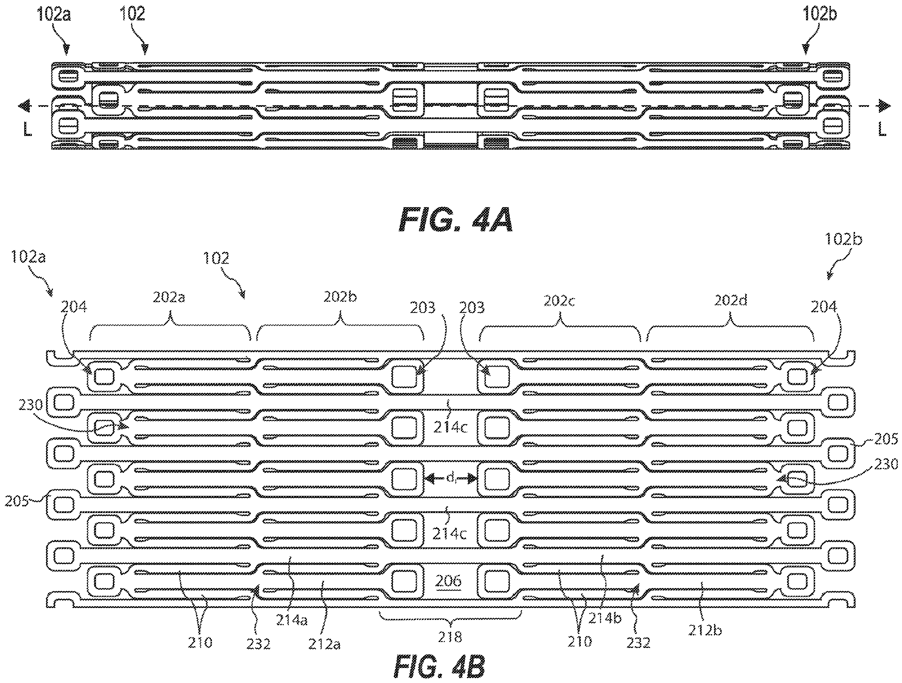

FIGS. 4A and 4B depict an example reinforcing element 102 for use with the treatment systems 100 of the present technology, shown in tubular and laid flat configurations, respectively. As demonstrated by FIGS. 4A and 4B, in some embodiments the reinforcing element 102 may comprise a mesh (such as a stent) having a generally tubular structure that surrounds a lumen extending between open longitudinal ends. The reinforcing element 102 may have a first end portion 102a, a second end portion 102b, and a length extending therebetween along the longitudinal axis L of the reinforcing element 102.

While the views provided in several of the figures provided herein show expandable devices laid flat for ease of explanation and understanding, the devices can be formed into a tubular shape. Also, as used herein, the term "longitudinal" can refer to a direction along an axis that extends through the lumen of the device while in a tubular configuration, and the term "circumferential" can refer to a direction along an axis that is orthogonal to the longitudinal axis and extends around the circumference of the device when in a tubular configuration.

According to some embodiments, for example as shown in FIGS. 4A and 4B, the mesh can comprise a plurality of struts 210 within a plurality of strut sections 202a-202d (referred to collectively as "strut sections 202"), a plurality of first spines 212 (labeled individually as 212a and 212b) and a plurality of second spines 214 (labeled individually as 214a, 214b, and 214c) extending substantially longitudinally across and between the strut sections 202, a plurality of first outer eyelets 205, a plurality of second outer eyelets 204, a plurality of opposing eyelets 203, and a plurality of junctions 230 and 232. The first and second spines 212 and 214 are collectively referred to herein as "spines 216." Only selected ones of the struts, spines, eyelets, and junctions have been labeled in FIG. 4B for ease of illustration. In some embodiments, all of the strut sections 202 and the spines 216 can extend along some or all of a circumference of the reinforcing element 102 when the reinforcing element 102 forms a tubular shape (i.e., its treatment shape).

In some embodiments, for example as shown in FIG. 4B, each end of one, some, or all of the struts 210 can be connected to a spine 216. For example, a first end of all of the struts 210 in in all of the strut sections 202 connects to a first spine 212 and a second end of all of the struts 210 in all of the strut sections 202 connect to a second spine 214. In some embodiments, a first end of one, some, or all of the struts 210 in one, some, or all of the strut sections 202 connects to a first spine 212 and a second end of one, some, or all of the struts 210 in one, some, or all of the strut sections 202 connect to a second spine 214. In some embodiments, the first and second ends of one, some, or all of the struts 210 in one, some, or all of the strut sections 202 connects to a first spine 212. In some embodiments, the first and second ends of one, some, or all of the struts 210 in one, some, or all of the strut sections 202 connect to a second spine 214. The struts 210 may connect to the first and/or second spines 212, 214 at a strut-eyelet junction 230 or at a strut junction 232 (only a few labeled). As depicted, the struts 210 can be arranged in a serpentine pattern where the struts 210 change longitudinal directions after crossing a spine. Some or all of the junctions can be formed at longitudinal ends of a strut section 202.

As previously mentioned, the mesh of the reinforcing element 102 may include first and second spines 212, 214 that extend longitudinally along the mesh. In some embodiments, all or a portion of one, some, or all of the first spines 212 can be parallel to all or a portion of one, some, or all of the second spines 214. In some embodiments, all or some of one, some, or all of the first spines 212 can be parallel to all or a portion of one, some, or all of the other first spines 212. In some embodiments, all or some of one, some, or all of the second spines 214 can be parallel to all or a portion of one, some, or all of the other second spines 214. In some embodiments, such as that shown in FIG. 4B, the first and second spines 212, 214 alternate around a circumference of the mesh. In some embodiments, at least one first spine 212 is directly radially adjacent at least one other first spine 212, and in some embodiments, at least one second spine 214 is directly radially adjacent at least one other second spine 214.

As shown in FIG. 4B, a first spine 212 may extend across less than all of the strut sections 202, and a second spine 214 may extend across all of the strut sections 202. The mesh may include an intermediate region 218 comprising opposing eyelets 203 and bridging portions 214c of the second spines 214. The first spines 212 may not extend into the intermediate region 218 and thus may form two circumferential sections 212a and 212b that are spaced apart along the longitudinal axis of the mesh. In some embodiments, one, some, or all of the first spines 212 may span only two strut sections 202. Within the intermediate region 218, a periphery of each of the opposing eyelets 203 and the bridging portions 214c of the second spines 212 may enclose a gap 206. The opposing longitudinal ends of the opposing eyelets 203 may be separated a distance di across the gap 206. In some instances, one, some, or all of the first spines 212 extend between second outer eyelets 204 and the adjacent one of the opposing eyelets 203. The first and second longitudinal ends of one, some, or all of the second spines 214 may terminate at second outer eyelets 205.

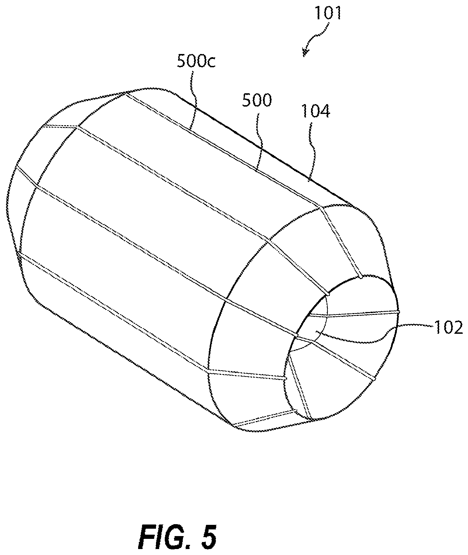

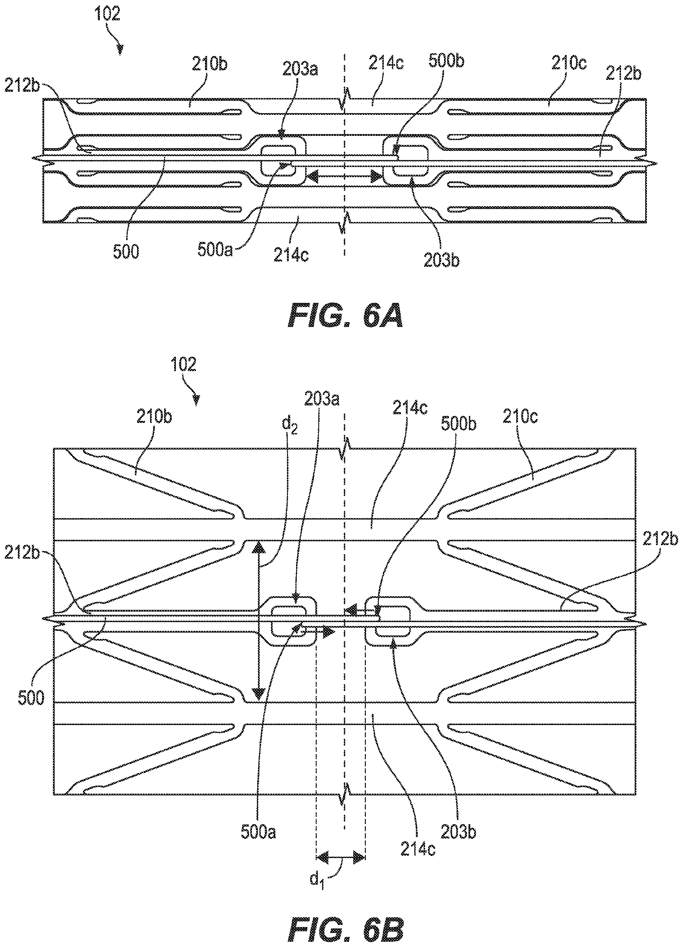

FIG. 5 is an isolated view of one example of a treatment element 101, shown in an expanded configuration, where the treatment element 101 includes the reinforcing element 102 shown in FIGS. 4A and 4B. The reinforcing element 102 is shown schematically in FIG. 5 for ease of illustration. FIG. 6A is an enlarged view of a portion of the reinforcing element 102, depicted as positioned in a collapsed state within the lumen of the expandable element 104. With reference to FIGS. 5 and 6A, in some embodiments the reinforcing element 102 may be coupled to the expandable element 104 via one or more connectors 500. The connector 500 may comprise at least one of a strand, a suture, a wire, a thread, a tether, a fiber, a filament, or other suitable connecting member. Each connector 500 may have a first portion 500a coupled to the reinforcing element 102 at a first location along the reinforcing element 102, a second portion 500b coupled to the reinforcing element 102 at a second location along the reinforcing element 102, and an intermediate portion 500c (FIG. 5) extending between the first and second portions 500a and 500b. In some embodiments, the first location is an eyelet 203a of an opposing eyelet pair, and the second location is the other eyelet 203b of the opposing eyelet pair.

The intermediate portion 500c of the connector 500 may extend over a radially outer surface of the expandable element 104, as shown in FIG. 5. In some embodiments, all or a portion of the length of the intermediate portion 500c may extend substantially longitudinally along the expandable element 104. As shown in FIG. 5, in some embodiments a plurality of connectors 500 can be spaced apart along the radially outer surface of the expandable element 104. In some embodiments, each of such connectors 500 can be separately coupled to the reinforcing element 102 at separate or overlapping locations. For example, each connector 500 may be coupled to a separate eyelet or other structure of the reinforcing element 102, or two or more connectors 500 may be coupled to the same eyelet or other structure of the reinforcing element 102.

In some embodiments, such as that shown in FIGS. 4A-6B, the first location is positioned at a longitudinal location along the reinforcing element between the first end and the second location, and the connector extends from the second location past the first location to the first end, then inverts and extends along an outer surface of the expandable element to the second end of the reinforcing element, then inverts and extends along the reinforcing element towards the second end past the second location to the first location.

In use, as the expandable element 104 is inflated, the outer wall of the expandable element 104 is pushed radially outwardly which pushes the intermediate portion 500c of the connector(s) 500 radially outwardly or radially away from the reinforcing element 102. As a result, the first portion 500a of the connector 500 pulls the corresponding eyelet (203a in FIG. 6B) in a first longitudinal direction and the second portion 500b of the connector 500 pulls the corresponding eyelet (203b in FIG. 6B) in a second longitudinal direction opposite the first longitudinal direction such that a distance di between the opposing eyelets 203a and 203b decreases. During this time the first spines 212 are also being pulled longitudinally by their corresponding eyelet 203, which puts a stress at the junctions 230, 232, thereby causing the struts 210 to flex at their joints and angle away from the adjacent spines. As the struts 210 swing out from the spines, the struts 210 push radially adjacent spines away from one another, thereby increasing the distance d₂ between the spines (as shown in FIG. 6B) and increasing a diameter of the reinforcing element 102.

As the connectors 500 pull the opposing eyelets 203 in opposite directions, the circumferential sections 212a, 212b of the first spines 212 move longitudinally towards one another, thereby shortening a length of the mesh as measured between the second outer eyelets 204. The second spines 214, however, do not change in length. As such, the reinforcing element 102 has selective foreshortening along its length.

Passing a flexible connector through the eyelets makes the eyelets act as pulleys and thereby reduces the force required to effect expansion. Using the eyelets as pulleys additionally can be advantageous for increasing the length of connector that must be pulled in order to expand the reinforcing element 102, which can be advantageous to provide more flexibility in the interaction between the expandable element 104 and the reinforcing element 102.

In some instances, the reinforcing element 102 may include a travel limiting element that prevents the reinforcing element 102 from radially expanding beyond a predetermined diameter and/or from lengthening beyond a predetermined length. For example, the reinforcing element could be designed with stops or elements which interfere once the reinforcing element 102 has opened to an appropriate diameter. In the examples shown in FIGS. 4A-6B, the connector portions passed through the opposing eyelets limit radial expansion of the reinforcing element 102. When the opposing eyelets 203 are pulled together and bump into each other, the expansion limit has been reached.

In some embodiments, the second outer eyelets 204 can be used as fairleads to keep the individual connectors 500 oriented and spaced appropriately as they wrap around the expandable element 104. For example, a particular connector could pass through an eyelet at one end of the reinforcing element 102, then through the more distant opposing eyelet, then through the other opposing eyelet, and then through the second outer eyelet 204 at the opposite end of the reinforcing element 102. Additionally or alternatively, the connector 500 can make more than one loop through the two opposing eyelets 203, creating additional force when pulling the opposing eyelets 203 together and making it easier for the expandable element 104 to expand the reinforcing element 102. The example reinforcing element 102 shown in FIGS. 4A-6B also provides the benefit of not needing to foreshorten; as previously discussed, only select spines need to foreshorten in order to effect stent expansion. This can be helpful with manufacturing and bonding to adjacent catheter and expandable element 104 structures.

The treatment element 101 of the present technology provides several advantages over the prior art. For example, because the treatment element 101 and/or expandable element 104 has a perfusion lumen when expanded, the expandable element 104 can remain expanded at the treatment site for an extended period of time (i.e., a minute or more). As a result, the treatment elements of the present technology provide more effective dilation of the stenosed native valve and of the prosthetic stent-valve being delivered, thereby reducing the likelihood of perivalvular leakage or stent slippage. The extended expansion time also reduces the possibility of incomplete or non-circular stent expansion, which can lead to valve leakage and/or increased valve deterioration and early valve failure. Additionally, the treatment elements of the present technology enable the clinician to dilate the valve more slowly or leave the valve partially dilated for a period of time, which improves the final positioning of the prosthetic valve as it improves targeting of position and depth of the valve deployment.

To avoid acute ventricular overexpansion and provide meaningful blood flow to the systemic circulation, the perfusion lumen may have a cross-sectional area of about 0.5 cm² to about 0.8 cm². A perfusion lumen of this size in the convention devices faces several challenges. First, for a catheter having such a large fixed lumen, it would not be possible to introduce it through a percutaneous sheath. Secondly, such a large perfusion lumen running through the center of the balloon would also be likely to collapse under the high pressure of the balloon unless the perfusion lumen is highly reinforced.

When the expandable element 104 is deflated, the decreasing fluid pressure within the expandable element 104 will allow the outer wall of the expandable element 104 to decrease, thereby allowing the shoulders of the expandable element 104 to elongate and flatten as the reinforcing element 102 naturally resumes its elongated, low-profile state.

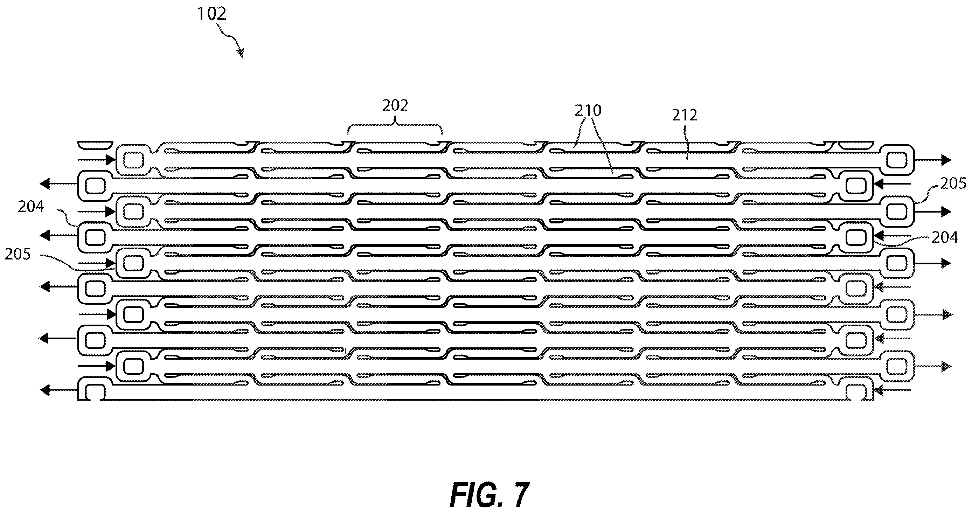

FIG. 7 depicts another example of a reinforcing element 102 for use with the treatment systems 100 of the present technology. The reinforcing element 102 of FIG. 7 is similar to the reinforcing element 102 of FIGS. 4A-6B, except: (a) the reinforcing element 102 of FIG. 7 does not have an intermediate region 216 with opposing eyelets 203, and (b) each of the spines has a first outer eyelet 204 at one end and a second outer eyelet 205 at the other end. To expand the reinforcing element 102, one or more connectors 500 may be threaded through or coupled to some or all of the first outer eyelets 205 such that the connector(s) extends from a first outer eyelet 205 at one end of the reinforcing element 102 and across the outer wall of the expandable element 104 to a first outer eyelet 205 at the other longitudinal end of the reinforcing element 102. As the expandable element 104 expands, the first outer eyelets are pulled longitudinally, thereby causing radial expansion of the reinforcing element 102. In contrast to the reinforcing element 102 of FIGS. 4A-6B, the reinforcing element 102 can change in its overall length as it expands. Additionally or alternatively, a compressive longitudinal force could be applied to the second outer eyelets 204 to radially expand the reinforcing element 102.

The reinforcing element 102 may be attached to the expandable element 104 at the ends of the reinforcing element 102. For example, the reinforcing element 102 may have fingers (not shown) extending from the ends of the reinforcing element 102 which are folded back against the outside of the reinforcing element 102. These fingers may be bonded to the ends of the expandable element 104, perhaps between the two tubes which form the expandable element. Alternatively, the fingers might have eyelets on both ends, and reinforcing connectors might be threaded back and forth between the eyelets at opposite ends over the outside of the expandable element. Therefore, the expandable element would be trapped between the stent on the inside, and the connectors on the outside. To avoid damaging the vessel wall or the heart valve (if it is being used for delivery of a valve), a second layer of expandable element material might be placed over these reinforcing connectors, or they might be bonded to the outside of the expandable element with adhesive or polymer.

As was the case with the reinforcing element 102 of FIGS. 4A-6B, the reinforcing element 102 of FIG. 7 may include a travel limiting element that prevents the reinforcing element 102 from radially expanding beyond a predetermined diameter and/or from lengthening beyond a predetermined length. For example, as depicted in FIG. 7, the reinforcing element 102 may include a flexible connector (e.g., a fiber, a suture, etc.) that loops through the first outer eyelets 705, thereby coupling the second outer eyelets 705 together. The connector in this case may have a length equivalent to the circumferential length of a desired expanded outer diameter of the reinforcing element 102. When the reinforcing element 102 expands to the preset diameter or length, the connector will prevent additional expansion. Additionally or alternatively, the first outer eyelets 205 could be coupled to first and/or second adjacent eyelets 204, 205 in such a fashion as to limit foreshortening, and thereby limit expansion of the outer diameter.

In some aspects of the technology, the expandable element 104 may be longer than the reinforcing element 102 and expansion of the reinforcing element 102 occurs via elongation of the reinforcing element 102. The reinforcing element 102 of FIG. 7, for example, could be used in such a manner. The expandable element 104 could be attached to first outer eyelets 205 of the reinforcing element 102. This attachment could be a direct connection by fusing the eyelets or other features with the wall or material comprising the expandable element 104 (such as a polymer), or again via connectors 500 that have been laced through the eyelets of the reinforcing element 102 and around the expandable element 104 to the eyelets at the other end of the reinforcing element 102.

Many of the embodiments disclosed herein utilize the force of the expandable element's expansion to expand the reinforcing element 102, even though expansion of the reinforcing element 102 reduces a volume of the expandable element 104 to some degree. For example, if the reinforcing element 102 were 30 mm long and had a relaxed diameter of 4 mm, and the reinforcing element 102 was then expanded to a diameter of 10mm to create a perfusion lumen, then a volume of the reinforcing element 102 would increase by approximately 2.0 cm³. If the expandable element 104 surrounding the reinforcing element 102 is initially inflated with an interior diameter of 4mm and the interior diameter expands to 10 mm, then a volume of the expandable element 104 would be reduced by that same 2 cc of volume. Therefore, in some embodiments it may be advantageous for the expandable element 104 to elongate as the reinforcing element 102 expands to create more volume than it is losing via the expansion of the reinforcing element 102, or the pressure of the expandable element 104 may keep the reinforcing element 102 deflated. For example, in an example where the expandable element 104 has an outer diameter of 26 mm and an interior diameter of 10 mm, elongation of the expandable element 104 by 8 mm would add approximately an additional 3.6 cm³ of volume. Therefore, if the reinforcing element 102, the expandable element 104, and the connector 500 arrangement is designed so that the expandable element 104 elongates by 8 mm as the reinforcing element 104 expands, then the system should preferentially cause the reinforcing element 104 to expand as the expandable element 104 is pressurized.

**In** some aspects of the technology, the perfusion lumen 108 may have a valve, such as a one-way valve, positioned at a location along its length. FIGS. 8A and 8B, for example, show an example treatment system 100 including a valve 800. In some embodiments, it may be especially beneficial to position the valve 800 at the proximal end of the expandable element 104, as shown in FIGS. 8A and 8B. **In** the example shown in FIG. 8A, the portion of the expandable element 104 surrounding the perfusion lumen 108 is extended proximally to form a short wind-sock valve which opens to permit blood to flow from the distal end of the catheter to the proximal end due to differential blood pressure. When the pressure proximally is higher than the pressure distally, the valve flattens against the elongated member 112 and impedes flow. Alternatively, if the expandable element 104 is being placed in such a way that the desired direction of flow is proximally to distally, the valve could be reversed and placed on the distal end of the expandable element 104 such that flow only proceeds distally.

The treatment system 100 may additionally or alternatively include other types of valves, such as an iris valve, a multi-leaflet valve, a duckbill valve, and others. Moreover, the valve 800 may be positioned at the distal end of the expandable element 104 or at any location along the length of the expandable element 104.

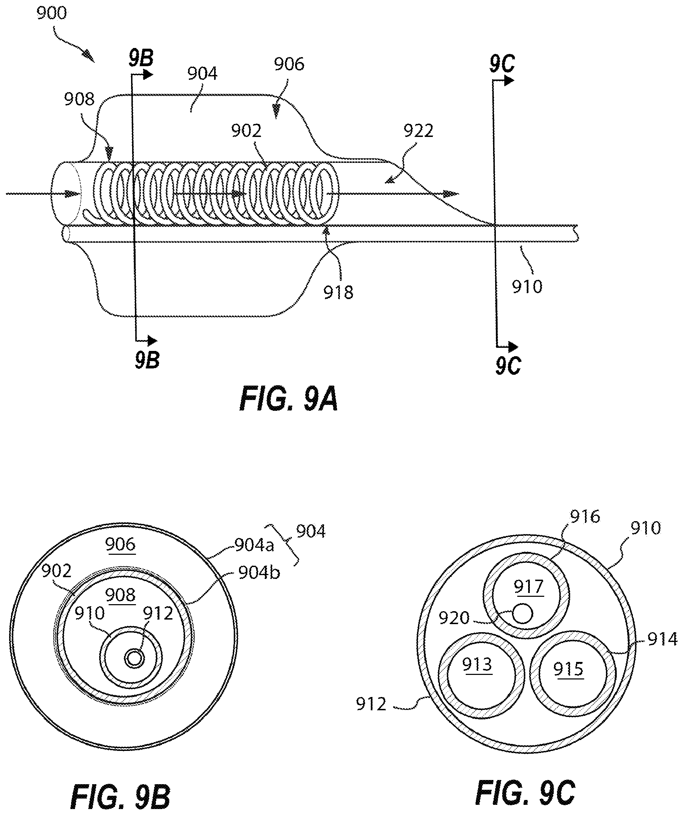

FIGS. 9A-10B illustrate additional embodiments of a treatment system 900 in accordance with the present technology. FIG. 9A is a cross-sectional side view of the distal portion of the treatment system 100, FIG. 9B is a cross-sectional view taken along line 9B-9B, and FIG. 9C is a cross-sectional view taken along line 9C-9C. Referring to FIGS. 9A-9C together, the treatment system 100 may comprise an expandable element 904 (e.g., a balloon) carried by the distal portion of an elongated carrier shaft 910. The expandable element 904 and the distal portion of the carrier shaft 910 may be configured to be intravascularly and/or percutaneously positioned at a treatment site at or within a blood flow passage. The expandable element 904 may have a collapsed configuration (not shown) for delivery to the treatment site and an expanded configuration (as shown in FIG. 9A) in which the expandable element 904 defines a perfusion lumen 908 therethrough. As a result, when the expandable element 904 is expanded within the blood flow passage, the expandable element 904 allows blood flow through its lumen 908. A reinforcing element 902 can be disposed within the lumen 908 of the expandable element 904 and configured to maintain patency of the lumen 908.

The carrier shaft 910 may have at least three generally tubular elongated members 912, 914 and 916 extending at least a portion of its length therethrough. For example, the carrier shaft 910 may include: (a) a first elongated member 912 defining a first lumen 913 therethrough (such as a guidewire lumen) for atraumatic guidance of the catheter to the appropriate location; (b) a second elongated member 914 having defining a second lumen 915 (such as an inflation lumen) for inflation and deflation of the expandable element 904 with saline, contrast medium, or other appropriate fluid; and (c) a third elongated member 916 defining a third lumen 917 therethrough for delivery of a reinforcing element 902 (discussed in greater detail below) to the lumen 908 of the expandable element 904. In some embodiments, the three lumens 913, 915, 917 can be formed as lumens within a unitary carrier shaft 910, rather than as lumens defined by separate tubular members disposed within a common lumen of the carrier shaft 910. In some embodiments, the carrier shaft 910 may have more or fewer shafts or lumens (e.g., one shaft, two shafts, four shafts, five shafts, etc., and/or one lumen, two lumens, four lumens, five lumens, etc.).

The carrier shaft 910 may extend through the entire length of the perfusion lumen 908 and terminate distal to a distal end of the expandable element 904, and/or the reinforcing element 102. In some embodiments, the carrier shaft 910 may terminate at a location that is longitudinally aligned with or proximal of a distal end of the treatment element 101, the expandable element 104, and/or the reinforcing element 102. In other embodiments, the carrier shaft 910 may terminate at a more proximal location, with some or all of the elongated members 912, 914 916 extending distally beyond a distal end of the carrier shaft 910.

The first elongated member 912 can extend substantially the entire length of the carrier shaft 910 and be configured to slidably receive a guidewire through the first lumen 913. The second elongated member 914 defining the second lumen 915 may extend between a proximal end portion (not shown) and a distal end portion that is fluidically coupled to the interior region 906 of the expandable element 904. For example, the carrier shaft 910 may have an opening in its sidewall to connect the second lumen 915 of the second elongated member 914 to the interior region 906 of the expandable element 904 (between the first and second tubes 904a, 904b). The second lumen 915 defined by the second elongated member 914 may be configured to deliver a fluid (e.g., saline, contrast solution) to the expandable element 904 to expand the expandable element 904. In some embodiments, second lumen 915 of the second elongated member 914 may be connected to the interior region 906 of the expandable element 904 via a separate tube.

The third elongated member 916 defining a third lumen 917 may extend between a proximal end portion (not shown) and a distal end portion that terminates adjacent a proximal end portion of the expandable element 904. In operation, a reinforcing element 902 (e.g., a wire) may be advanced through the third lumen 917, out of an opening 918 in the carrier shaft 910, and into the lumen 908 of the expandable element 904, as described in more detail below. For example, third lumen 917 may terminate at the opening 918 formed in a sidewall of the carrier shaft 910. The opening 918 may face towards the perfusion lumen 908 of the expandable element 904, at or near a proximal end portion of the expandable element 904. To facilitate advancement through the lumen 917, the reinforcing element 918 may be coupled to an elongated pusher element 920. In some embodiments, the pusher element 920 can be an elongated shaft or rod extending through the lumen 917 and coupled at its distal end to a proximal end of the reinforcing element 902, such that distally advancing the pusher element 920 causes the reinforcing element 902 to be distally advanced within the lumen 917.

In some embodiments, the expandable element 904 may be made from two extruded tubes, such as first and second tubes 904a and 904b, thereby defining an interior region 906. In such embodiments, the first tube 904a may be expanded to form the outer surface of the expandable element 904, and the second tube 904b may be used to form the inner lumen of the expandable element 904. The first and second tubes 904a and 904b may be welded together at their respective proximal and distal ends to form the expandable element 904. The first elongated member 912 may extend between these two expandable elements and into the interior region 906. The tubes of the expandable element 904 may be welded around the carrier shaft 910 at the proximal and distal ends of the expandable element 904. In some embodiments, the expandable element may be formed of an inverted tube similar to that described above with respect to expandable element 204, with the first elongated member 912 extending into the interior region 906 defined by the expandable element.

The expandable element 904 may be made from one or more materials commonly used for balloons used in interventional procedures, such as polyethylene, polyolefin, polyurethane, nylon (polyamide), polyethylene terephthalate (PET or polyester), and/or other suitable polymers. The expandable element may also be a composite structure which may contain any polymer or polymers plus reinforcing fibers such as Kevlar, carbon-fiber, spectra or other high molecular weight polyethylenes, or other fibers in any orientation.

In some aspects of the technology, the perfusion lumen 908 may have a valve, such as a one-way valve, positioned at a location along its length. As shown in FIG. 9A, for example, the treatment system 900 including a valve 922. In some embodiments, it may be especially beneficial to position the valve 922 at the proximal end of the expandable element 904. In some embodiments, the portion of the expandable element 904 surrounding the perfusion lumen 908 extends proximally to form a short wind-sock valve which opens to permit blood to flow from the distal end of the treatment system 900 to the proximal end due to differential blood pressure. When the pressure proximally is higher than the pressure distally, the valve flattens against the carrier shaft 910 and impedes flow. Alternatively, if the expandable element 904 is being placed in such a way that the desired direction of flow is proximally to distally, the valve 922 could be reversed and placed on the distal end of the expandable element 904 such that flow only proceeds distally.

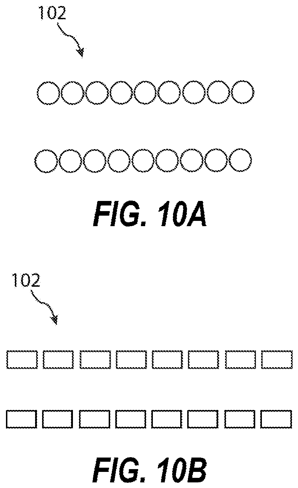

In some embodiments, the reinforcing element 902 may extend through at least a portion of the perfusion lumen 908. As shown in FIG. 9A, for example, the reinforcing element 902 can be a wire which is pre-formed to take the shape of a relatively closely wound coil, approximately the size of the perfusion lumen 908. Advancing the wire through the third lumen 917 of the third elongated member 916 causes the wire to be deployed into the perfusion lumen 908 where it resumes its coiled shape (shown in FIG. 9A) and reinforces the lumen 908. The wire may be distally advanced further into the perfusion lumen 908 until a distal end of the wire is at or near a distal portion of the expandable element 904. The wire may be round in cross-section (e.g., FIG. 10A), or generally square (e.g., FIG. 10B), or relatively ribbon-shaped, depending on the size and shape of the third lumen 917 and the number of coils which are desired to be deployed to reinforce the perfusion lumen 908. The wire can be metallic, polymeric, or any other suitable material. In some embodiments, the wire can be a shape-memory material (e.g., nitinol).

In some embodiments, to retrieve the system 900, the reinforcing element 902 (e.g., a coiled wire) may be proximally retracted into the lumen 917 of the third elongated member 916. Once the reinforcing element 902 has been retracted, the expandable element 904 may be deflated (e.g., by applying negative pressure via the second lumen 915 of the second elongated member 914), and the system 900 may be retracted into a catheter or otherwise removed from the treatment site.

In some embodiments, the reinforcing element could be a braid which is pre-formed to resume a relatively flat, close-wound tube when it is deployed into the perfusion lumen 908. Such a braided reinforcing element 902 can be attached at a distal end to the carrier shaft 910 and at a proximal end portion to a pusher element (not shown). When the pusher element is distally advanced relative to the carrier shaft 910, the reinforcing element 902 is foreshortened and urged to assume an expanded state. In this expanded state, the braid may provide an increased outward radial force to maintain patency of the perfusion lumen 908 and/or expand the expandable element 904. This may be advantageous in embodiments where it is preferred to expand the reinforcing element 902 at a separate time from expansion of the expandable element 904. For example, using such a configuration, the pusher element could be used to expand the reinforcing element before, concurrently with, or after expansion of the expandable element (e.g., via inflation through the second lumen 915).

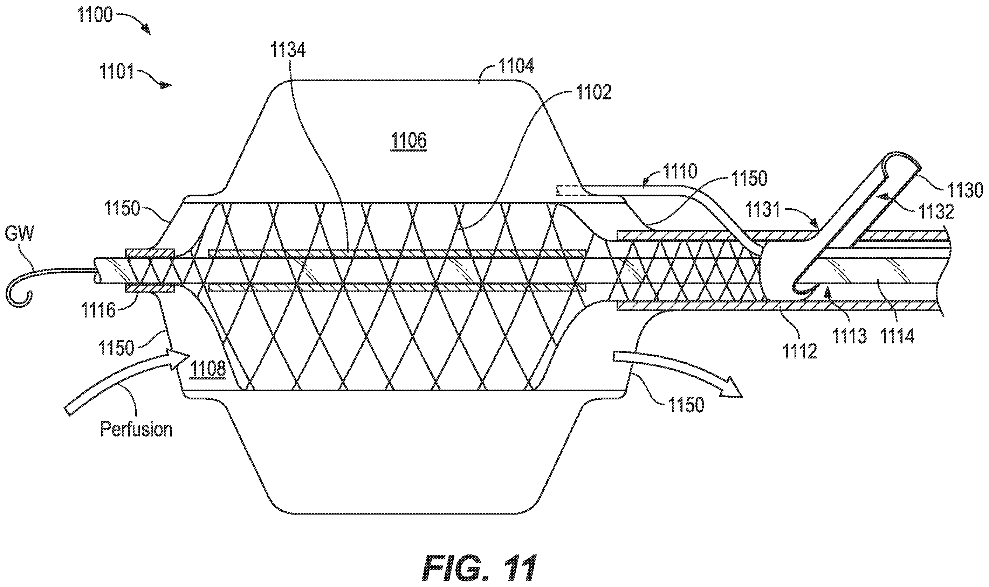

FIG. 11 is a schematic, cross-sectional side view of a distal portion of a treatment system 1100 of the present technology, shown in an expanded configuration. As shown in FIG. 11, the treatment system 1100 may comprise a treatment element 1101 configured to be intravascularly and/or percutaneously positioned at a treatment site at or within a blood flow passage. The treatment element 1101 may comprise a braided reinforcing element 1102 and an expandable element 1104 (e.g., a balloon). The expandable element 1104 may have a collapsed configuration (not shown) for delivery to the treatment site and an expanded configuration (as shown in FIG. 11) in which the expandable element 1104 defines a perfusion lumen 1108 therethrough. As a result, when the expandable element 1104 is expanded within the blood flow passage, the expandable element 1104 allows blood flow through its lumen 1108. The reinforcing element 1102 can be disposed within the lumen 1108 of the expandable element 1104 and configured to generate a radial force sufficient to maintain patency of the lumen 1108. As shown in FIG. 11, the reinforcing element 1102 may be a tubular braid or weave.

The treatment system 1100 may further comprise one or more elongated members. For example, the treatment system 1100 may comprise a first elongated member 1110 defining a first lumen therethrough (not visible in the drawings), a second elongated member 1114 defining a second lumen 1115 therethrough, which might be used to deliver a guidewire GW in advance of the rest of the system, and a third elongated member 1112 defining a third lumen 1113 therethrough. The first elongated member 1110 may extend between a proximal end portion (not shown) and a distal end portion that is fluidically coupled to the interior region 1106 of the expandable element 1104. For example, a distal end portion of the first elongated member 1110 may be inserted into and/or placed into fluid communication with the interior region 1106 of the expandable element 1104 via a seam between the outer and inner walls at a proximal end portion of the treatment element 1101. The first elongated member 1110 may be configured to deliver a fluid (e.g., water, saline, a radiopaque solution, air) to the expandable element 1104 to expand the expandable element 1104.

The second elongated member 1114 may extend between a proximal end portion (not shown) and a distal end portion located at or near the treatment element 1101. The second elongated member 1114 can be a generally tubular shaft defining a lumen 1115 therethrough. In some embodiments, the second elongated member 1114 is configured to slidably receive a guidewire GW for atraumatic guidance of the catheter to the appropriate location. The second elongated member 1114 may extend through the entire length of the perfusion lumen 1108 and terminate distal to a distal end of the treatment element 1101, the expandable element 1104, and/or the reinforcing element 1102. In some embodiments, the second elongated member 1114 may terminate at a location that is longitudinally aligned with or proximal of a distal end of the treatment element 1101, the expandable element 1104, and/or the reinforcing element 1102. In some embodiments, the second elongated member 1114 may have an atraumatic distal tip at its distal end (such as that shown in FIGS. 2A-2D).

A distal portion of the reinforcing element 1102 may be fixed to the second elongated member 1114 while a proximal portion of the reinforcing element 1102 may be configured to move axially relative to the second elongated member 1114. This way, movement of the proximal portion of the reinforcing element 1102 distally and proximally causes the reinforcing element to expand and contract, respectively.

The third elongated member 1112 may comprise a generally tubular shaft defining a lumen 1113 therethrough. The third elongated member 1112 may extend from a proximal portion (not shown) to a distal portion that terminates at a location longitudinally aligned with or proximal of the treatment element 1101, the expandable element 1104, and/or the reinforcing element 1102. In some embodiments, the third elongated member 1112 terminates at a location longitudinally aligned with or distal to a proximal end of the treatment element 1101, the expandable element 1104, and/or the reinforcing element 1102. In some embodiments, the third elongated member 1112 extends through the entire length of the treatment element 1101, the expandable element 1104, and/or the reinforcing element 1102.

The third elongated member 1112 may receive one or more elongated members (including tubular shafts) through its lumen 1113. For example, as shown in FIG. 11, the first elongated member 1114 may extend through the lumen 1113 of the third elongated member 1112. In some embodiments, the first elongated member 1110 may also extend through the lumen 1113 of the third elongated member 1112. In some embodiments, the treatment system 1100 may have more or fewer elongated members or lumens than that depicted in FIG. 11.

In some embodiments, the third elongated member 1112 may have an opening 1131 (e.g., a side port) through its sidewall, and the treatment system 1100 may include a pusher 1130 extending from the proximal portion of the treatment system 1100 to the distal portion, through the opening 1131, and terminating at or proximate the proximal end of the reinforcing element 1102. In some embodiments, at least a portion of the length of the pusher 1130 may have a hollow, hemispherical shape surrounding an elongated opening 1132 so that a distal portion of the pusher 1130 can slide over the second elongated member 1114. A distal terminus of the pusher 1130 may have a complete tubular shape (as shown in FIG. 11). In some embodiments, the side port 1131 may be disposed at the proximal hub, and at varying distances more distally along the shaft up to adjacent to the expansion element 1104. According to several embodiments, the pusher 1130 does not exit lumen 1113 through a sidewall of the third elongated member 1112 and instead remains within the lumen 1113.

A distal end of the pusher 1130 may abut a proximal end of the reinforcing element 1102, either directly or indirectly via a coupler (not shown). In any case, distal movement of the pusher 1130 pushes the proximal end of the reinforcing element 1102 distally, thereby causing more of the length of the reinforcing element 1102 to exit the lumen 1113 of the third elongated member 1112 into the lumen 1108 of the expandable element 1104. As the proximal portion of the reinforcing element 1102 is pushed distally (while the distal portion is held stationary), the portion of the reinforcing element 1102 released into the lumen 1108 radially expands into apposition with an inner surface of the expandable element 1104. The reinforcing element 1102 may be expanded within the lumen 1108 of the expandable element 1104 while the expandable element 1104 is deflated, partially inflated, or fully inflated.

A distal end portion of the treatment element 1101 may include a coupler 1116 that surrounds a distal end portion of the reinforcing element 1102. For example, the coupler 1116 may be positioned around the reinforcing element 1102 to secure the distal end portion of the reinforcing element 1102 to the second elongated member 1114.

In some embodiments, the expandable element 1104 may be coupled at its proximal end portion to a distal portion of the third elongated member 1112, and may be coupled at its distal end portion to the coupler 1116 and/or the second elongated member 1114. As shown in FIG. 11, the expandable element 1104 may be coupled to one of more portions of the treatment element 1101 directly or indirectly via one or more couplers. In some embodiments, the expandable element 1104 is only coupled at one of its distal or proximal portions. In those embodiments that include one or more couplers, the coupler may comprise a rigid member. In some embodiments, the coupler comprises a flexible member. For example, the coupler may comprise one or more tethers 1150 extending between the expandable element 1104 and the coupler 1116 and/or the second elongated member 1114. The tethers 1150 can comprise a suture, filament, wire, thread, or other flexible connecting structure. In any embodiment, the coupler can have a first end fixed to the expandable element 1104 (e.g., fixed to only the inner tube, fixed to only the outer tube, fixed to the inner and the outer tube, fixed between the inner and out tube, etc.) and the other end fixed at coupler 1116 and/or second elongated member 1114. The treatment element 1104 can include a single coupler or a plurality of couplers spaced apart about a circumference of the expandable element 1104. In some embodiments, the couplers have slack when the expandable element 1104 and/or reinforcing element 1102 are in a low-profile state, and may be substantially taut when the expandable element 1104 and/or reinforcing element 1102 are in an expanded state. The couplers can have a length sufficient for the reinforcing element 1102 to expand into opposition with an inner surface of the expandable element lumen.

According to some embodiments, the treatment element 1101 includes a stopper 1134 positioned within the lumen of the reinforcing element 1102 between the coupler 1116 and the pusher 1130 such that the stopper 1134 may limit the amount of axial compression exerted on the reinforcing element 1102. In some embodiments, a distal end of the stopper 1134 may be fixed to the coupler 1116 or the proximal end of the stopper 1134 may be fixed to the pusher 1130.

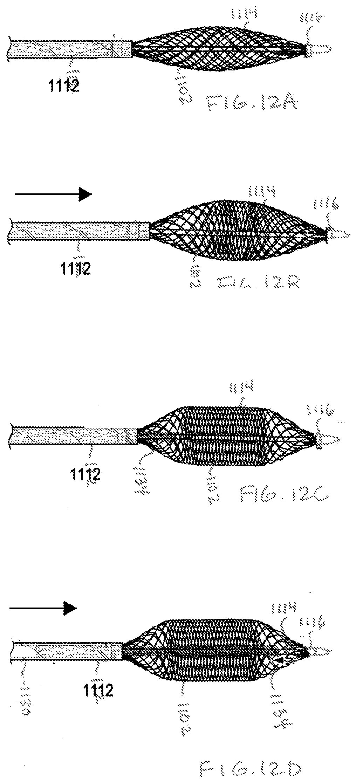

FIGS. 12A-12D depict deployment of the reinforcing element 1102. The expandable element 1104 has been removed in FIGS. 12A-12D so that the effect of axial movement of the pusher 1130 on the length and cross-sectional dimension of the reinforcing element 1102 can be better visualized. It will be appreciated that when the reinforcing element 1102 is positioned within the lumen 1108, the shape and/or extent of the radial expansion of the reinforcing element 1102 will be constrained to some extent by the cross-sectional dimension of the lumen 1108.

As shown in FIGS. 12A-12D, the pusher 1130 can be moved distally relative to the second elongated member 1114, thereby causing radial expansion of the reinforcing element 1102. In some embodiments, the second elongated member 1114 is pulled proximally relative to the pusher 1130 to expand the reinforcing element 1102. As the pusher 1130 pushes a proximal end portion of the reinforcing element 1102 distally, a greater length of the reinforcing element 1102 is released from the second elongated member 1112. The newly-introduced material of the reinforcing element 1102 axially compresses the already-exposed portion of the reinforcing element 1102, thereby increasing a braid density (and thus radial outward force) of the reinforcing element 1102. Advancement of the pusher 1130 also advances the stopper 1134, which as previously mentioned can limit the amount of axial compression exerted on the reinforcing element 1102.

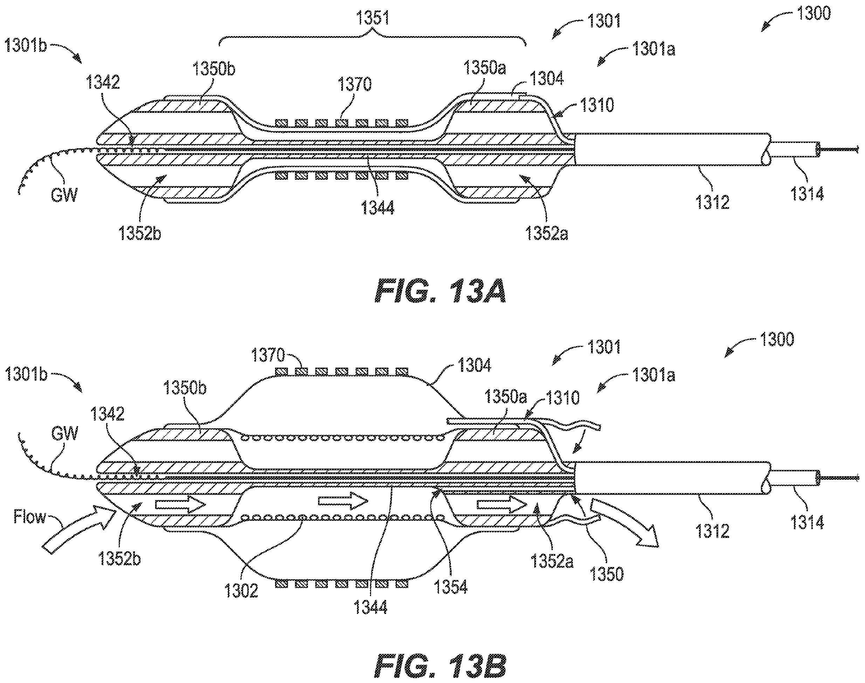

FIGS. 13A and 13B show a distal portion of a treatment system 1300 configured in accordance with the present technology. The treatment system 1300 comprises a treatment element 1301 configured to be deployed within a blood flow passage at a treatment site. The treatment element 1301 may have a collapsed state (FIG. 13A) and an expanded state (FIG. 13B), and may comprise an expandable implantable valve apparatus, stent, or other implantable device 1370 positioned around an expandable element 1304 (such as a balloon). In some embodiments, the treatment element 1301 does not include an expandable implant 1370.

The treatment system 1300 may include a catheter 1340 having one or more lumens. The treatment element 1301 can be carried by a distal portion of the catheter 1340. The catheter 1340 may include a guidewire lumen 1342 that is configured to slidably receive a guidewire GW. The guidewire lumen 1342 can be integral with a body of the catheter 1340 or can be defined by a separate shaft extending through a lumen of the catheter 1340 or axially along an outer surface of the catheter 1340.

The expandable element 1304 can be coupled to the distal portion of the catheter 1340. For example, a first, radially-inner portion of the expandable element 1304 can be coupled to the catheter 1340 (for example, along one or both of the annular shoulders 1350a, 1350b described herein). In some embodiments the catheter 1340 includes an inflation lumen 1310 fluidically coupled to an interior region 1306 of the expandable element 1304. The inflation lumen 1310 can be integral with a body of the catheter 1340 or can be defined by a separate shaft extending through a lumen of the catheter 1340 or axially along an outer surface of the catheter 1340.

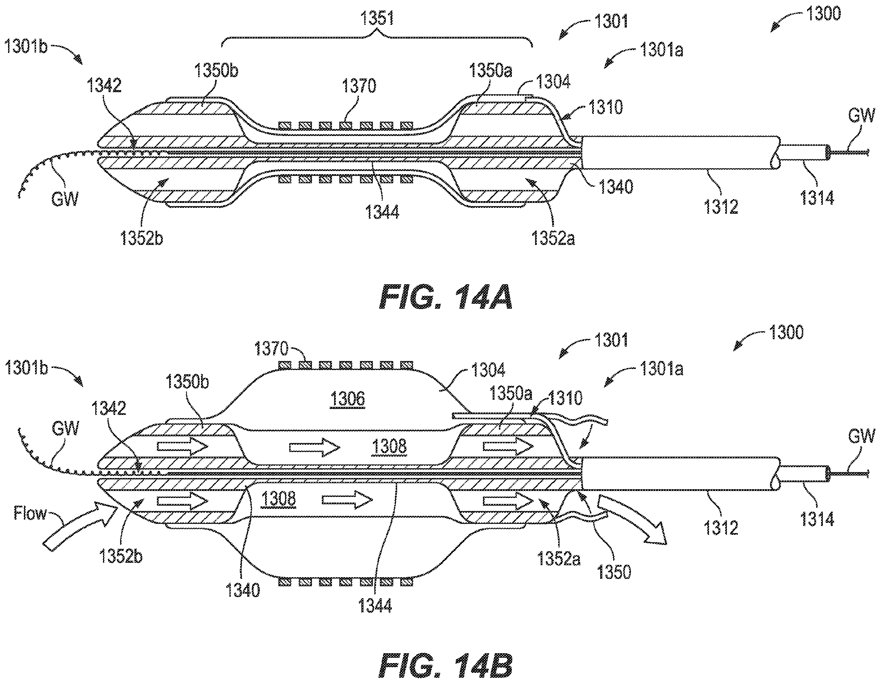

In some embodiments, for example as shown in FIGS. 13A and 13B, the catheter 1340 includes a lumen 1354 for introduction of one or more reinforcing elements 1302. The reinforcing elements 1302 may comprise one or more wires, braids, or smaller longitudinal tubular balloons. Before, during, or after inflation of the expandable element 1304, the reinforcing elements 1302 could be expanded to create or maintain a perfusion lumen 1308 through the center of the annular expandable element 1304. As shown in FIGS. 14A and 14B, in some embodiments the treatment system 1300 and/or treatment element 1301 does not include a reinforcing element 1302. In such embodiments, the catheter 1340 would not include lumen 1342.

As shown in FIGS. 13A and 13B, in some embodiments the catheter 1340 may include proximal and distal annular shoulders 1350a and 1350b, respectively, connected by a narrowed region 1344. An outer diameter of the catheter 1340 at the narrowed region 1344 can be less than an outer diameter of the catheter 1340 at each of the proximal and distal annular shoulders 1350a and 1350b. In some embodiments, the proximal shoulder 1350a, the distal shoulder 1350b, and the narrowed region 1344 together comprise the distal portion 1351 of the catheter 1340. One or both of the inner and outer tubes of the expandable element 1304 may be attached to the annular shoulders 1350a, 1350b, and the shoulders 1350a, 1350b may have one or more lumens 1352a, 1352b, respectively, therethrough. The lumens 1352a, 1352b may also be annular (as shown), or may extend around less than a full circumference of the corresponding shoulders 1350a, 1350b.

For example, the distal annular shoulder 1350b may have one or more distal lumens 1352b, and the proximal annular shoulder 1350a may have one or more proximal lumens 1352a. A diameter of the catheter 1340 along the narrowed region 1344 may be less than the average diameter of the catheter 1340 along each of the shoulder regions 1350a, 1350b. When the expandable element 1304 is in an expanded state (as shown in FIG. 13B), the proximal and distal catheter lumens 1352a, 1352b may fully or at least partially align with the perfusion lumen 1308 of the expandable element 1304 such that blood may flow through the perfusion lumen 1308 during the procedure.

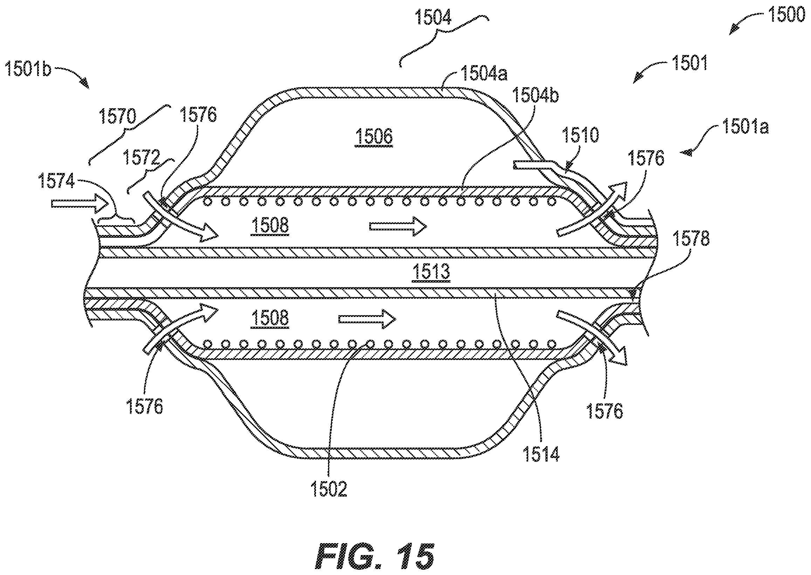

FIG. 15 is a schematic cross-sectional side view of a distal portion of a treatment system 1500 configured in accordance with the present technology. The treatment system 1500 can comprise a treatment element 1501 at its distal portion. As shown in FIG. 15, in some embodiments the treatment element 1501 can include one or more annular shoulders 1572 formed in whole or in part of the expandable element 1504. For example, the expandable element 1504 can comprise an outer tube 1504a and an inner tube 1504b, and the end portions of one or both tubes 1504a, 1504b can define one or both shoulders. For example, the end portions of the inner and outer tubes 1504a, 1504b can be fixed together (e.g., with a coupler, adhesive, fusion, welding, etc.) along end regions 1570 (only one labeled for ease of viewing). The combined ends create a stiffer region which can be deformed into a curved, tapered shape. A first portion 1572 of the end regions 1570 can be coupled to an elongated shaft 1514, and a second portion 1574 of the end regions 1570 can be bent away from the shaft 1514 to form proximally and distally facing surfaces 1572 at the proximal and distal portions 1501a, 1501b, respectively, of the treatment element 1501. The proximally and distally facing surfaces 1572 can comprise one or more openings 1576 to allow blood to flow through the flow lumen 1508 defined between the expandable element 1504 (and/or reinforcing element 1502) and the elongated shaft 1514. While FIG. 15 shows the treatment element 1501 including a reinforcing element 1502 between the expandable element 1504 and the elongated shaft 1514, in some embodiments the treatment element 1501 does not include a reinforcing element 1502. In those embodiments including a reinforcing element 1502, the reinforcing element 1502 can comprise any of the reinforcing elements disclosed herein (e.g., a laser-cut stent, a braid, a coil, etc.). In any of these embodiments, the reinforcing element 1502 can be delivered to the distal portion of the treatment system 1500 via a lumen of the treatment system 1500, such as through lumen 1513 of the elongated shaft 1514 or through a separate lumen, such as lumen 1578.

The treatment element 1501 may have a collapsed state (not shown) and an expanded state (FIG. 15), and may comprise an expandable implantable valve apparatus, stent, or other implantable device (not shown) positioned around an expandable element 1304 (such as a balloon). In some embodiments, the treatment element 1501 does not include an expandable implant.

In those embodiments for use in a valve replacement procedure (such as TAVR), any of the treatment systems disclosed herein may be configured for use with an expandable implantable valve apparatus (not shown). In some embodiments, the valve apparatus may be preloaded around an outer surface of the expandable element such that the valve apparatus and the treatment element are delivered to the native valve annulus together. When expanded at the treatment site, the treatment element pushes radially outwardly against an inner surface of the valve apparatus, thereby forcing the valve apparatus to radially expand into apposition with the annular tissue. In some embodiments, the valve apparatus may already be expanded at the native valve annulus and the treatment element may be delivered to an interior region of the valve apparatus and expanded to further expand or secure the valve apparatus at the annulus. In some embodiments the valve apparatus comprises only a laser-cut stent, and in some embodiments the valve apparatus comprises a laser-cut stent and a prosthetic heart valve configured for implantation at a native valve annulus.

### Conclusion

Although many of the embodiments are described above with respect to systems, devices, and methods for treating cardiac disease, the technology is applicable to other applications and/or other approaches, such as pulmonary or cerebral applications. Moreover, other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described above with reference to FIGS. 2A-15.

The above detailed descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Where a range of values is provided, it is understood that each intervening value, to the tenth of the unit of the lower limit unless the context clearly dictates otherwise, to between the upper and lower limits of that range is also specifically disclosed. Each smaller range between any stated value or intervening value in a stated range and any other stated or intervening value in that stated range is encompassed within the disclosure. The upper and lower limits of these smaller ranges may independently be included or excluded in the range, and each range where either, neither or both limits are included in the smaller ranges is also encompassed within the disclosure, subject to any specifically excluded limit in the stated range. Where the stated range includes one or both of the limits, ranges excluding either or both of those included limits are also included in the disclosure.

## Claims

1. A system (1100) for treating a blood flow passage of a patient, the system comprising:
a treatment element (1101) comprising:
an expandable element (1104) configured to be positioned within the passage, the expandable element having a collapsed configuration and an expanded configuration in which the expandable element defines a lumen (1108) therethrough, and
a reinforcing element (1102) positioned within the lumen of the expandable element, the reinforcing element having a collapsed configuration and an expanded configuration in which the reinforcing element defines a lumen therethrough;
a pushing element (1130) comprising a proximal end portion and a distal end portion, wherein the distal end portion is configured to engage a proximal end portion of the reinforcing element such that distal movement of the pushing element causes the reinforcing element to radially expand;
a first elongated member (1110) defining a first lumen therethrough, the first lumen in fluid communication with an interior region of the expandable element, wherein the first elongated member is configured to deliver a fluid to the interior region of the expandable element to expand the expandable element;
a second elongated member (1114) defining a second lumen (1115) therethrough, wherein a distal portion of the second elongated member is coupled to a distal portion of the reinforcing element; and
a third elongated member (1112) defining a third lumen (1113) therethrough, the third lumen configured to receive the first and second elongated members and the pushing element therein.

2. The system of Claim 1, wherein the distal end portion of the pushing element is configured to substantially abut the proximal end portion of the reinforcing element.

3. The system of any one of Claims 1 to 2, wherein, distal movement of the proximal end portion of the reinforcement element causes the reinforcing element to axially compress and radially expand.

4. The system of any one of Claims 1 to 3, wherein the distal end portion of the pushing element has a tubular shape.

5. The system of any one of Claims 1 to 4, wherein the pushing element exits the third lumen through an opening (1131) in a sidewall of the third elongated member.

6. The system of any one of Claims 1 to 5, wherein the reinforcing element is configured to contact an inner wall of the expandable element.

7. The system of any one of Claims 1 to 6, wherein the distal portion of the second elongated member is coupled to the distal portion of the reinforcing element via a coupler.

8. The system of any one of Claims 1 to 7, further comprising a stopper (1134) configured to limit axial translation of the pushing element and/or axial compression of the reinforcing element.

9. The system of Claim 8, wherein the stopper is positioned proximal of the coupler.

10. The system of Claim 9, wherein a distal end portion of the stopper is secured to the coupler.

11. The system of Claim 8, wherein a proximal end portion of the stopper is secured to the pushing element.

12. The system of any one of Claims 1 to 11, wherein the expandable element is secured to the second elongated member and/or the third elongated member by a tether.

## Patentansprüche

1. System (1100) zum Behandeln eines Blutflussdurchgangs eines Patienten, wobei das System ein Behandlungselement (1101) umfasst, das Folgendes umfasst:
ein expandierbares Element (1104), das konfiguriert ist, um innerhalb des Durchgangs positioniert zu werden, wobei das expandierbare Element eine kollabierte Konfiguration und eine expandierte Konfiguration aufweist, in der das expandierbare Element ein Lumen (1108) dadurch definiert, und
ein Verstärkungselement (1102), das innerhalb des Lumens des expandierbaren Elements positioniert ist, wobei das Verstärkungselement eine kollabierte Konfiguration und eine expandierte Konfiguration aufweist, in der das Verstärkungselement ein Lumen dadurch definiert;
ein Schubelement (1130), das einen proximalen Endabschnitt und einen distalen Endabschnitt umfasst, wobei der distale Endabschnitt konfiguriert ist, um mit einem proximalen Endabschnitt des Verstärkungselements in Eingriff zu kommen, sodass eine distale Bewegung des Schubelements eine radiale Ausdehnung des Verstärkungselements bewirkt;
ein erstes längliches Element (1110), das ein erstes Lumen dadurch definiert, wobei das erste Lumen in Fluidverbindung mit einem Innenbereich des expandierbaren Elements steht, wobei das erste längliche Element konfiguriert ist, um ein Fluid in den Innenbereich des expandierbaren Elements zu leiten, um das expandierbare Element zu expandieren;
ein zweites längliches Element (1114), das ein zweites Lumen (1115) dadurch definiert, wobei ein distaler Abschnitt des zweiten länglichen Elements mit einem distalen Abschnitt des Verstärkungselements gekoppelt ist; und
ein drittes längliches Element (1112), das ein drittes Lumen (1113) dadurch definiert, wobei das dritte Lumen konfiguriert ist, um das erste und das zweite längliche Element und das Schubelement darin aufzunehmen.

2. System nach Anspruch 1, wobei der distale Endabschnitt des Schubelements konfiguriert ist, um im Wesentlichen an den proximalen Endabschnitt des Verstärkungselements anzugrenzen.

3. System nach einem der Ansprüche 1 bis 2, wobei eine distale Bewegung des proximalen Endabschnitts des Verstärkungselements bewirkt, dass das Verstärkungselement sich axial komprimiert und radial expandiert.

4. System nach einem der Ansprüche 1 bis 3, wobei der distale Endabschnitt des Schubelements eine röhrenförmige Form aufweist.

5. System nach einem der Ansprüche 1 bis 4, wobei das Schubelement das dritte Lumen durch eine Öffnung (1131) in einer Seitenwand des dritten länglichen Elements verlässt.

6. System nach einem der Ansprüche 1 bis 5, wobei das Verstärkungselement konfiguriert ist, um eine Innenwand des expandierbaren Elements zu berühren.

7. System nach einem der Ansprüche 1 bis 6, wobei der distale Abschnitt des zweiten länglichen Elements über eine Kupplung mit dem distalen Abschnitt des Verstärkungselements gekoppelt ist.

8. System nach einem der Ansprüche 1 bis 7, das ferner einen Stopfen (1134) umfasst, der konfiguriert ist, um die axiale Verschiebung des Schubelements und/oder die axiale Kompression des Verstärkungselements zu begrenzen.

9. System nach Anspruch 8, wobei der Stopfen proximal zu der Kupplung positioniert ist.

10. System nach Anspruch 9, wobei ein distaler Endabschnitt des Stopfens an der Kupplung befestigt ist.

11. System nach Anspruch 8, wobei ein proximaler Endabschnitt des Stopfens am Schubelement befestigt ist.

12. System nach einem der Ansprüche 1 bis 11, wobei das erweiterbare Element durch eine Leine an dem zweiten länglichen Element und/oder dem dritten länglichen Element befestigt ist.

## Revendications

1. Système (1100) destiné à traiter un passage de flux sanguin d'un patient, le système comprenant un élément de traitement (1101) comprenant :
un élément extensible (1104) conçu pour être positionné dans le passage, l'élément extensible ayant une configuration repliée et une configuration déployée dans laquelle l'élément extensible définit une lumière (1108) à travers celui-ci, et
un élément de renforcement (1102) positionné dans la lumière de l'élément extensible, l'élément de renforcement ayant une configuration repliée et une configuration déployée dans laquelle l'élément de renforcement définit une lumière à travers celui-ci ;
un élément de poussée (1130) comprenant une partie d'extrémité proximale et une partie d'extrémité distale, dans lequel la partie d'extrémité distale est conçue pour s'engager dans une partie d'extrémité proximale de l'élément de renforcement de sorte que le mouvement distal de l'élément de poussée provoque l'expansion radiale de l'élément de renforcement ;
un premier élément allongé (1110) définissant une première lumière à travers celui-ci, la première lumière étant en communication fluidique avec une région intérieure de l'élément extensible, dans lequel le premier élément allongé est conçu pour délivrer un fluide à la région intérieure de l'élément extensible afin d'étendre l'élément extensible ;
un deuxième élément allongé (1114) définissant une deuxième lumière (1115), dans lequel une partie distale du deuxième élément allongé est couplée à une partie distale de l'élément de renforcement ; et
un troisième élément allongé (1112) définissant une troisième lumière (1113) à travers celui-ci, la troisième lumière étant conçue pour recevoir les premier et deuxième éléments allongés et l'élément de poussée à l'intérieur.

2. Système selon la revendication 1, dans lequel la partie d'extrémité distale de l'élément de poussée est conçue pour s'appuyer sensiblement sur la partie d'extrémité proximale de l'élément de renforcement.

3. Système selon l'une quelconque des revendications 1 à 2, dans lequel le mouvement distal de la partie d'extrémité proximale de l'élément de renforcement entraîne une compression axiale et une expansion radiale de l'élément de renforcement.

4. Système selon l'une quelconque des revendications 1 à 3, dans lequel la partie d'extrémité distale de l'élément de poussée a une forme tubulaire.

5. Système selon l'une quelconque des revendications 1 à 4, dans lequel l'élément de poussée sort de la troisième lumière par une ouverture (1131) dans une paroi latérale du troisième élément allongé.

6. Système selon l'une quelconque des revendications 1 à 5, dans lequel l'élément de renforcement est conçu pour entrer en contact avec une paroi intérieure de l'élément extensible.

7. Système selon l'une quelconque des revendications 1 à 6, dans lequel la partie distale du second élément allongé est couplée à la partie distale de l'élément de renforcement par l'intermédiaire d'un coupleur.

8. Système selon l'une quelconque des revendications 1 à 7, comprenant en outre une butée (1134) conçue pour limiter la translation axiale de l'élément de poussée et/ou la compression axiale de l'élément de renforcement.

9. Système selon la revendication 8, dans lequel la butée est positionné à proximité du coupleur.

10. Système selon la revendication 9, dans lequel une partie d'extrémité distale de la butée est fixée au coupleur.

11. Système selon la revendication 8, dans lequel une partie d'extrémité proximale de la butée est fixée à l'élément de poussée.

12. Système selon l'une quelconque des revendications 1 à 11, dans lequel l'élément extensible est fixé au deuxième élément allongé et/ou au troisième élément allongé par une attache.
